# NOUVEAU FASCICULE DE BREVET EUROPEEN

(11) **EP 3 282 837 B2**
(45) Date de publication et mention de la décision concernant l'opposition: **14.01.2026**
(45) Mention de la délivrance du brevet: 06.01.2021
(21) Numéro de dépôt: 16731201.6
(22) Date de dépôt: 13.04.2016
(51) Int. Cl.: A01K 67/033, B65D 19/00, B65G 57/00, B65G 1/00

(54) **ATELIER D'ÉLEVAGE D'INSECTES**
FARM ZUR AUFZUCHT VON INSEKTEN
FARM FOR REARING INSECTS

(30) Priorité: 13.04.2015 FR 1553207
(43) Date de publication de la demande: 21.02.2018
(60) Demande divisionnaire: 20213781.6
(73) Titulaire: Ynsect, 91058 Evry-Courcouronnes Cedex (FR)
(72) Inventeur: COMPARAT, Solène, 91000 Evry (FR); HUBERT, Antoine, 94140 Alfortville (FR); BERRO, Fabrice, 75001 Paris (FR); LEVON, Jean-Gabriel, 75011 Paris (FR); LAUNAY, Franck, 77850 Hericy (FR); SARTON DU JONCHAY, Thibault, 60710 Chevrières (FR)
(74) Mandataire: Santarelli
(86) Numéro de dépôt international: PCT/FR2016/050849
(87) Numéro de publication internationale: WO 2016/166471

(56) Documents cités:
- WO-A1-2014/171829
- US-A- 5 819 685
- US-A1- 2011 238 207

## Description

La présente invention concerne le domaine de l'élevage d'insectes. En particulier, elle porte sur un atelier ou unité d'élevage d'insectes.

Les insectes, en particulier certaines espèces, peuvent constituer une source de produits ou de matières premières, notamment pour l'alimentation animale ou humaine, ou à destination de nombreuses autres industries.

Sauf indication contraire, le terme « insecte » employé dans le présent document désigne tout stade d'évolution de l'œuf ou oothèque à l'insecte adulte, en passant par la larve et la nymphe ou pupe.

En particulier, le terme « larve » désigne dans le présent document le stade larvaire des insectes, ce qui inclut l'asticot pour les diptères et la chenille pour les lépidoptères, ainsi que les stades aptères chez les orthoptères. Le terme « nymphe » désigne dans le présent les stades intermédiaires entre la larve et l'imago, ce qui inclut la pupe pour les diptères, la nymphe pour les coléoptères, la chrysalide pour les lépidoptères et, le cas échéant, un stade intermédiaire au cours duquel certaines modifications physiologiques (prépupe) ou comportementales des individus apparaissent, telles qu'une sclérification importante de la cuticule pour les diptères. De la même manière, le terme « œuf » couvre également une oothèque de dictyoptères.

Typiquement, certaines espèces d'insectes comestibles sont riches en protéines. Environ deux mille espèces d'insectes comestibles ont été identifiées à ce jour, et ce nombre croît régulièrement. De nombreux insectes peuvent être employés pour l'alimentation des animaux d'élevage terrestres (mammifères, oiseaux...), des poissons et invertébrés aquatiques d'élevage, etc. Les insectes convertissent de manière générale une grande proportion de ce qu'ils ingèrent en masse corporelle (notamment nettement plus que les mammifères). En effet, ils ont un métabolisme d'organismes poïkilothermes qui ne nécessite pas d'utiliser de l'énergie pour maintenir leur température corporelle. A contrario, les animaux supérieurs, dits homéothermes, emploient une énergie importante pour maintenir leur température corporelle. La domestication des insectes à des fins de production alimentaire constitue ainsi une opportunité vis-à-vis des enjeux mondiaux en matière de nutrition et de préservation de l'environnement.

Outre l'aspect alimentaire, les insectes peuvent constituer une ressource importante dans de nombreux domaines industriels. Typiquement, l'exosquelette des insectes est constitué en grande partie de chitine, dont un dérivé connu est le chitosan. Les applications de la chitine et/ou du chitosan sont nombreuses : cosmétique (composition cosmétique), médicale et pharmaceutique (composition pharmaceutique, traitement des brûlures, biomatériaux, pansements cornéens, fils chirurgicaux), diététique et alimentaire, technique (agent filtrant, texturant, floculant ou adsorbant notamment pour la filtration et dépollution de l'eau), etc. En effet, la chitine et /ou le chitosan sont des matériaux biocompatibles, biodégradables et non toxiques.

L'élevage des insectes connaît un certain essor. Certaines méthodes et certains dispositifs relatifs à un tel élevage ont ainsi été développés. On connaît par exemple au travers du document WO2014/171829 un procédé et un dispositif associé permettant d'automatiser l'apport en nourriture dans des caisses d'élevage d'insectes. Plus précisément, ce document divulgue un dispositif permettant de déterminer, par un procédé d'observation de chacune des caisses d'un élevage, l'état et le stade de croissance des insectes présents dans chacune des caisses, et si un apport de nourriture est nécessaire dans la caisse considérée.

Ainsi, si certains dispositifs connus répondent à certaines problématiques de simplification dans un élevage d'insectes, il n'est pas connu de dispositif et de procédé permettant d'envisager l'élevage d'insectes à grande échelle dans des conditions optimisées.

En particulier, les problématiques de logistique et de gestion d'un élevage à grande échelle restent inconnues et irrésolues dans l'état de la technique. Or, un élevage à grande échelle permettrait l'obtention de quantités de produits suffisantes pour intéresser les marchés de commodités alimentaires et chimiques notamment.

La présente invention a pour but de résoudre au moins l'un des inconvénients précités. La présente invention vise notamment à proposer un dispositif, en particulier un atelier, optimisant la logistique associée à l'élevage d'insectes.

En particulier, l'invention porte sur un atelier d'élevage d'insectes, comportant une première zone dans laquelle les insectes en cours d'élevage sont stockés au cours de leur croissance dans des contenants et une seconde zone comportant au moins un poste configuré pour la réalisation d'une opération d'élevage sur les insectes d'un contenant ou sur ledit contenant ; les contenants étant groupés dans la première zone en ensembles de contenants palettisés dits unités élémentaires, chaque unité élémentaire contenant lors de sa formation uniquement des insectes au même stade d'évolution la première zone comportant des rayonnages à palettes dans lesquels peuvent être disposées les unités élémentaires; la première zone étant en outre équipée d'un dispositif automatisé configuré pour le déplacement des unités élémentaires entre la première zone et une interface avec la seconde zone. L'interface est une zone de dépôt d'une unité élémentaire. L'interface est dotée d'un système de convoyage, le système de convoyage permettant l'envoi de l'unité élémentaire vers la seconde zone, ou ladite interface permettant de dé-palettiser et/ou dégrouper les contenants d'élevage de l'unité élémentaire et le système de convoyage permettant leur envoi vers la seconde zone.

La répartition en deux zones de l'atelier permet l'optimisation de nombreux aspects de l'élevage. La première zone met en œuvre un dispositif automatisé pour la récupération de palettes et l'envoi dans la seconde zone, de sorte qu'aucune ou presque aucune opération d'élevage n'est à mener dans la première zone. L'élevage organisé en palettes permet de regrouper et manipuler un nombre important de lots unitaires d'insectes, chaque lot étant constitué d'insectes au même stade de développement ou de croissance. En outre, il permet une grande automatisation, les palettes et contenants d'élevage étant aisément opérables par des robots ou automates. Cela permet des cadences élevées de production dans l'atelier. Un stockage sur palette des contenants d'élevage peut être mis en œuvre dans des rayonnages adaptés, ce qui permet une importante optimisation spatiale de la zone de stockage, dans les trois dimensions spatiales. La formation d'unités élémentaires d'élevage, comportant des insectes au même stade d'évolution, permet une gestion séquentielle simple du procédé d'élevage mis en œuvre dans l'atelier. L'atelier proposé dans l'invention permet donc d'adopter des procédés et une gestion de production de type industrielle jusqu'alors inconnue dans le domaine de l'élevage des insectes.

Dans un tel atelier d'élevage d'insectes le dispositif automatisé peut avantageusement comporter un transstockeur pouvant se déplacer le long de ou entre les rayonnages.

Le dispositif automatisé peut être adapté à se déplacer à l'intérieur des rayonnages.

Le dispositif automatisé peut par exemple comporter un transstockeur qui se déplace le long des rayonnages pour chercher les charges sur une profondeur d'un ou deux rayonnages. Il peut comporter un système de navette qui se déplace horizontalement (en profondeur, et le cas échéant en longueur et/ou largeur) à l'intérieur des rayonnages pour déposer et prendre des palettes lorsque plusieurs rayonnages sont juxtaposés en profondeur. La palette est récupérée peut être transférée par exemple à un transstockeur ou un ascenseur.

Les contenants peuvent notamment être des caisses empilables, les unités élémentaires comportant une pluralité de caisses empilées, en une ou plusieurs colonnes, sur une palette. Selon une autre variante, les unités élémentaires comportent un rack (ou système d'étagères) adapté à recevoir les contenants afin de former une ou plusieurs colonnes de contenants ;

Dans ce cas, une unité élémentaire peut notamment comporter une à quatre colonnes constituées chacune de quatre à vingt-cinq caisses. Une unité élémentaire peut alternativement comporter une à quatre colonnes constituées chacune de quatre à trente-cinq caisses.

Les unités élémentaires peuvent avoir une hauteur comprise entre 1,80m et 3m, et de préférence entre 2m et 2,80m. Par exemple, les unités élémentaires peuvent avoir une hauteur comprise entre 1,80m et 2,40m, et de préférence entre 2m et 2,20m.

Par ailleurs, les rayonnages peuvent être configurés pour le stockage de deux à quinze unités élémentaires en hauteur, et de une ou deux unités élémentaires en profondeur.

Les rayonnages peuvent être configurés pour le stockage de deux à vingt unités élémentaires en hauteur, et de une à vingt-deux unités élémentaires en profondeur

Selon un mode de réalisation, la première zone peut être divisée en silos, destinés au stockage de larves ou d'insectes à des stades de croissance différents et/ou d'espèces différentes, lesdits silos étant séparés par des moyens de cloisonnement

De manière avantageuse, l'atelier peut comporter un dispositif de contrôle d'au moins un paramètre environnemental parmi la température, l'hygrométrie de l'air, la pression atmosphérique, la lumière et sa périodicité, la teneur de l'air en oxygène, la teneur de l'air en composés organiques volatils, et la teneur de l'air en particules fines, configuré pour appliquer une valeur de paramètre environnemental différente à chaque ensemble de rayonnages.

Selon un mode de réalisation, la seconde zone peut comporter un système automatisé de convoyage pour le déplacement d'unités élémentaires ou de contenants dégroupés vers l'au moins un poste de ladite seconde zone.

La seconde zone peut notamment comporter un poste de dé-palettisation et dégroupage des contenants.

La seconde zone peut comporter un poste de groupage des contenants en unité élémentaire.

La seconde zone peut comporter une pluralité de postes, chaque poste étant configuré pour l'une ou plusieurs opérations d'élevage choisies parmi :
- le nourrissage ;
- l'apport d'eau ;
- la calibration, en taille, masse, volume ou densité des insectes ;
- le tri entre larves vivantes, mortes, et déjections ;
- le tri entre adultes vivants et adultes morts ;
- le tri entre nymphes vivantes et nymphes mortes ;
- le tri entre au moins deux stades d'évolution des insectes entre œufs, larves, nymphes, et adultes,
- la séparation des insectes vivants et du substrat non consommé ;
- le tri entre insectes et œufs ;
- l'ajout d'insectes dans un contenant d'élevage ;
- l'abattage ou la destruction d'insectes
- le lavage de contenants.

Un tel atelier peut notamment comporter un poste configuré pour la calibration, en taille, masse, volume ou densité des insectes adultes et/ou le tri entre larves vivantes, mortes, et déjections et/ou le tri entre insectes adultes et larves ou nymphes, comportant un dispositif de séparation selon la densité et la prise à l'air.

L'atelier peut comporter un poste configuré pour la calibration, en taille ou volume des larves vivantes et/ou le tri entre larves vivantes, mortes, insectes adultes vivants, morts, nymphes vivantes, mortes, œufs, substrat et déjections, comportant un dispositif de tri optique. Le tri optique peut permettre notamment le tri des insectes par taille, ou selon d'autres paramètres visuellement indentifiables, tels que la couleur, la forme, le mouvement, etc.

L'atelier peut comporter un poste configuré pour la calibration des larves vivantes et/ou le tri entre larves vivantes, mortes, insectes adultes vivants, morts, nymphes vivantes, mortes, œufs, substrat et déjections, comportant un élément vibrant tel qu'un tamiseur ou une table vibrante.

L'atelier peut comporter un poste configuré pour la calibration des larves vivantes et/ou le tri entre larves vivantes, mortes, insectes adultes vivants, morts, nymphes vivantes, mortes, œufs, substrat et déjections, comportant une table densimétrique.

L'atelier peut comporter un poste configuré pour la calibration des larves vivantes et/ou le tri entre larves vivantes, mortes, insectes adultes vivants, morts, nymphes vivantes, mortes, œufs, substrat et déjections, comportant une calibreuse à rouleaux.

L'atelier peut comporter un poste configuré pour la calibration des larves vivantes et/ou le tri entre larves vivantes, mortes, insectes adultes vivants, morts, nymphes vivantes, mortes, œufs, substrat et déjections, comportant un élément en rotation permettant l'éjection des larves par force centrifuge.

Les différents postes peuvent être alimentés par des dispositifs automatisés. Selon le poste considéré, il peut s'agir d'une alimentation automatisée en substrat, nourriture, eau, en contenant (pleins ou vides), en contenu de contenants vidés, etc.

L'atelier peut comporter un dispositif d'identification de caisses ou d'unités élémentaires adapté à être mis en œuvre par des moyens électroniques, ledit dispositif d'indentification comportant un système de radio-identification ou un système de communication par ondes.

L'atelier peut comporter en outre un ensemble de capteurs comportant :
- un capteur de masse configuré pour déterminer la masse d'une unité élémentaire ou d'un contenant ; et/ou
- un capteur de couleur, configuré pour déterminer la couleur des insectes, nymphe, ou œuf, du substrat, de l'eau, et/ou des déjections dans un contenant ; et/ou
- un capteur d'épaisseur ou de volume configuré pour déterminer l'épaisseur ou du volume de substrat dans un contenant ;
- un capteur de taille, configuré pour déterminer la taille des insectes, nymphe, ou œuf dans un contenant.

D'autres particularités et avantages de l'invention apparaîtront encore dans la description ci-après.

Aux dessins annexés, donnés à titre d'exemples non limitatifs :
- la figure 1 présente l'organisation générale d'un atelier conforme à un mode de réalisation de l'invention ;
- la figure 2 présente, selon une vue schématique en trois dimensions, un exemple d'atelier conforme à un mode de réalisation de l'invention ;
- La figure 3 présente, selon une vue schématique, une unité élémentaire pour l'élevage d'insectes ;
- La figure 4 présente un exemple d'organisation d'une première zone d'un atelier conforme à un mode de réalisation de l'invention ;
- La figure 5 présente un exemple d'organisation d'une première zone d'un atelier conforme à un autre mode de réalisation de l'invention
- La figure 6 présente, selon une vue schématique en trois dimensions, une première zone d'un atelier conforme à un mode de réalisation de l'invention adoptant l'organisation présentée à la figure 4.
- La figure 7 présente, selon une vue schématique en trois dimensions, une variante de la première zone présentée à la figure 6 ;
- La figure 8 présente un exemple d'organisation d'une première zone d'un atelier conforme un autre mode de réalisation de l'invention ;
- La figure 9 présente, selon une vue schématique, un exemple d'organisation d'une seconde zone d'un atelier conforme à un mode de réalisation de l'invention.

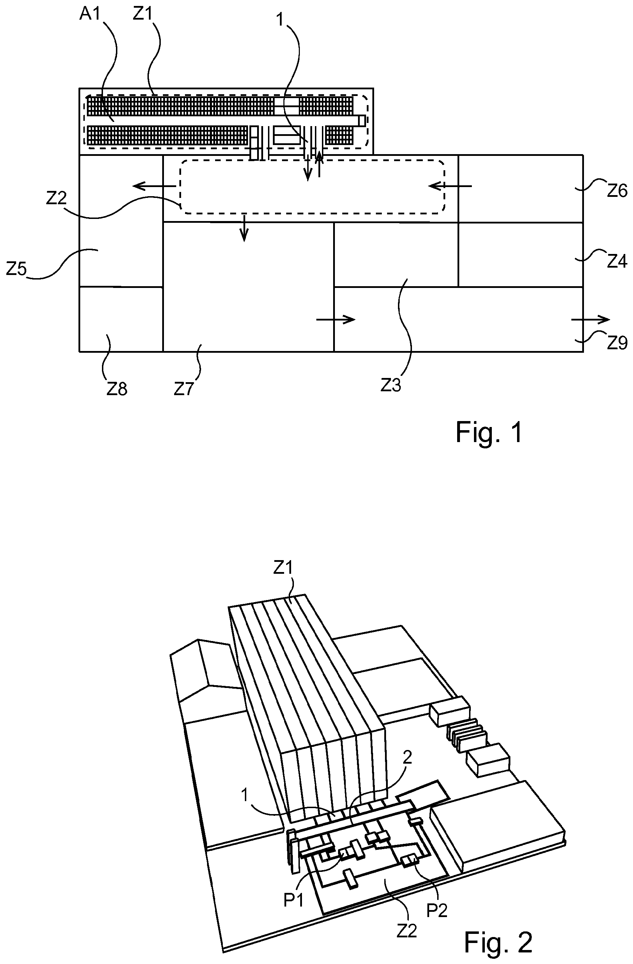

Tel que représenté à la figure 1, un atelier d'élevage d'insectes conforme à l'invention, ici représenté sous la forme d'un plan schématique vu de dessus, comporte au moins deux zones, à savoir une première zone Z1 organisée pour le stockage des insectes pendant leur croissance. Dans cette première zone Z1, les insectes grandissent dans des conditions environnementales (définies par des paramètres environnementaux dont la température, l'hygrométrie...) contrôlées et optimisées.

La notion d'élevage d'insectes comprend la croissance d'insectes adultes jusqu'à un stade désiré, mais peut également comprendre toutes les phases précédant l'obtention d'un insecte adulte, depuis la ponte des œufs, le développement de l'œuf, l'éclosion, le stade larvaire, la nymphose, le stade nymphal, l'émergence, etc. L'élevage d'insecte peut notamment être envisagé comme un ensemble organisé permettant la ponte d'œufs par des insectes adultes pour la production de larves, certaines larves étant élevées jusqu'au stade adulte pour la ponte de nouveaux œufs, les adultes étant renouvelés régulièrement (par exemple suite à leur mort) par des adultes jeunes assurant de nouvelles pontes, et ainsi de suite. Le produit final de la production peut être des œufs, et/ou des larves, et/ou des nymphes, et/ou des insectes adultes.

L'atelier comporte également une seconde zone Z2, organisée pour la réalisation d'une ou plusieurs opérations d'élevage. Les opérations d'élevage correspondent à des opérations devant être menées pour le maintien en vie, la bonne croissance, et/ou l'optimisation des conditions d'élevage des insectes. Il peut s'agir, entre autres opérations, de :
- nourrir les insectes ;
- leur apporter de l'eau ;
- renouveler le substrat dans lequel ils sont élevés ;
- les trier (pour permettre d'extraire les œufs, de séparer les insectes par taille, d'extraire les morts au cours de l'élevage, etc.) ;
- identifier les insectes présentant des symptômes de maladie ;
- densifier ou dé-densifier des unités élémentaires pour maximiser la production sans porter préjudice au bien-être et à la santé des insectes, afin d'optimiser la productivité de l'élevage ;
- abattre les insectes surnuméraires, malades, et/ou contaminés ou parasités ;
- ajouter de nouvelles souches d'insectes (afin de conserver la bonne santé de la lignée);
- conditionner les déjections pour leur valorisation.

Le substrat d'élevage, c'est-à-dire le milieu ajouté dans les contenants, adapté à la vie des insectes ou larves ou nymphes, pouvant contenir la nourriture destinée aux insectes ou larves ou nymphes peut se présenter sous forme d'un solide (particules, flocons, etc.) sec, d'un solide humide, ou d'un liquide.

La seconde zone Z2 comporte en particulier un ou plusieurs postes de travail spécialisés dans la réalisation d'une ou plusieurs séquences d'élevage. Une séquence d'élevage peut correspondre à une opération ou à un enchaînement de plusieurs opérations qui la constituent. La seconde zone Z2 peut être conformée pour permettre la mise en œuvre, à un poste ou plusieurs postes de travail, de séquences d'élevage constituées d'une succession d'opérations. Les postes de travail peuvent typiquement être regroupés en ilots pour la mise en œuvre d'opérations successives.

La figure 1 présente un plan d'organisation possible de la première zone Z1. Les insectes (œufs, larves, nymphes, ou adultes) sont élevés dans des contenants groupés en unités élémentaires d'élevage sous la forme de palettes. Les palettes sont stockées dans la première zone Z1 dans des rayonnages à palettes. Dans l'exemple ici représenté, les rayonnages à palettes sont séparés par une allée A1 permettant la circulation entre les rayonnages. Plusieurs ensembles parallèles de rayonnages/allées/rayonnages peuvent être définis pour constituer la zone Z1. Les rayonnages peuvent par exemple comprendre de 1 à 20 emplacements pour palette. Par exemple, l'allée A1 peut permettre la circulation d'un dispositif automatisé, typiquement un transstockeur, permettant de déplacer les unités élémentaire vers une interface 1 avec la seconde zone Z2. L'interface 1 est être une zone de dépôt d'une unité élémentaire, dotée d'un système de convoyage tel qu'un convoyeur à bande dont la mise en fonctionnement permet l'envoi de l'unité élémentaire vers la seconde zone 2 pour réalisation d'une opération d'élevage.

La figure 2 présente, selon une vue schématique en trois dimensions, un exemple d'atelier conforme à un mode de réalisation de l'invention. Dans l'exemple ici représenté, les rayonnages à palettes sont formés d'une structure poteaux-poutres dans laquelle des passages sont ménagés pour la circulation de transstockeurs entre les rayonnages. La seconde zone Z2 comporte un convoyeur à bande 2 permettant la circulation des unités élémentaires ou des contenants dégroupés dans ladite seconde zone Z2. Le convoyeur à bande 2 permet de manière générale la circulation dans la seconde zone Z2 depuis l'interface avec la première zone Z1 vers le ou les postes P1 et/ou P2, éventuellement groupés en ilots de postes, et dédiés à une ou plusieurs opérations d'élevage, et entre les postes. Le convoyeur à bande 2 peut permettre le retour de l'unité élémentaire ou des contenants vers l'interface I, ou, optionnellement, vers une seconde interface (non représentée) dédiée à la transition des unités élémentaires de la seconde zone Z2 vers la première zone Z1.

Pour la réalisation de certaines opérations, il peut s'avérer nécessaire de dé-palettiser et/ou dégrouper les contenants d'élevage. Cette opération peut, selon diverses organisations possibles, être réalisée au niveau de l'interface I, ou sur un poste dédié de la seconde zone Z2.

En plus de la première zone Z1 et de la seconde zone Z2, un atelier d'élevage d'insectes conforme à un mode de réalisation de l'invention peut comporter diverses zones complémentaires : une zone de préparation de produits d'élevage Z8, notamment des fluides, dont ceux permettant le contrôle des conditions atmosphériques de l'élevage, une zone d'entrée de nouvelle souches, une zone d'expéditions de souches.

D'autres zones complémentaires de l'élevage peuvent être présentes : une zone de laboratoire Z3, une zone de bureau Z4, une zone de traitement des déchets Z5, une zone de préparation de la nourriture d'élevage Z6, une zone de production de produits Z7, pour la production de divers produits à partir des insectes élevés, une zone logistique Z9, etc.

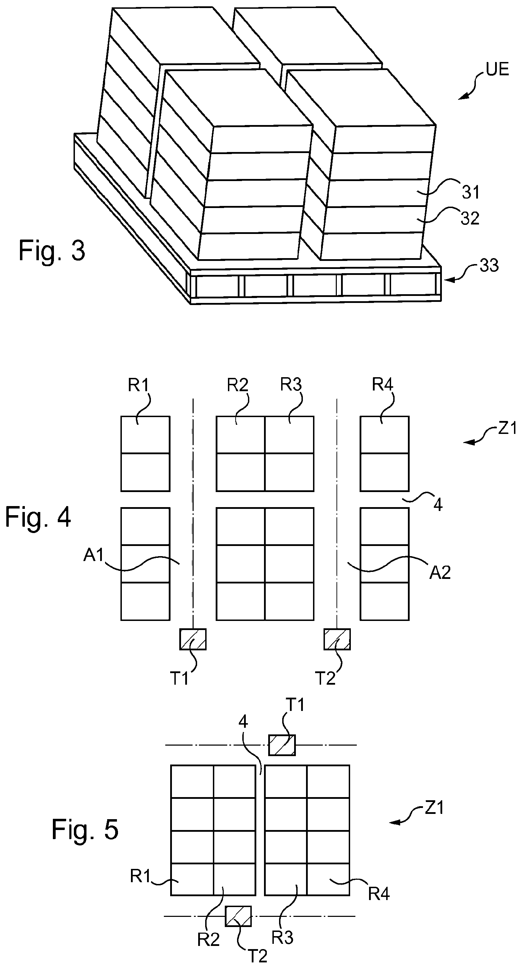

Dans l'invention, la croissance des insectes, c'est-à-dire les phases d'élevage en dehors des opérations ponctuelles d'élevage, s'effectue dans des unités élémentaires d'élevage. Il s'agit du groupement d'un ensemble de contenants d'élevage. La figure 3 illustre un exemple d'unité d'élevage, selon une représentation de principe en trois dimensions. En particulier, les contenants d'élevage peuvent être des caisses ou bacs empilables. Par bacs ou caisses empilables, on désigne en particulier des bacs ou caisses qui se superposent les unes au-dessus des autres, de manière légèrement encastrée, ce qui procure une certaine stabilité à la colonne de caisses ainsi formée.

Les caisses empilables sont réalisées en un matériau imputrescible. Elles peuvent notamment être réalisées en matériau plastique. De préférence, le matériau employé est du type alimentaire, c'est-à-dire un matériau autorisé pour le contact alimentaire.

Il peut s'agir notamment de caisses de géométrie simple, avec l'une ou plusieurs des caractéristiques suivantes : une forme générale parallélépipédique rectangle, un fond plat, des bords (parois latérales) verticaux. Elles peuvent comporter une face supérieure ouverte, notamment pour l'élevage de coléoptères, ou une face supérieure fermée de sorte à former une cage l'élevage, notamment pour l'élevage de diptères (avec, typiquement, des parois grillagées permettant le passage de l'air et de la lumière). Elles peuvent en outre être dotées de moyens d'encastrement correspondants entre la face supérieure et la face inférieure, tel que des pions destinés à se loger dans des alésages correspondants lors de l'empilement des caisses.

En outre, les caisses doivent présenter une résistance verticale suffisante pour supporter leur empilement et une bonne robustesse afin de résister à l'ensemble des manipulations, des nettoyages, et aux produits nettoyants afin de permettre leur réutilisation. Le nombre de caisses empilées est variable selon différents modes de réalisation de l'invention, et peut par exemple aller jusque vingt caisses par colonne de caisses. Chaque caisse peut par exemple présenter une charge utile (c'est-à-dire une masse pouvant être stockée dans la caisse) comprise entre 0,2 et 10kg, préférentiellement entre 0,5 et 5 kg et plus préférentiellement entre 1 et 3 kg. Typiquement, la charge d'une caisse peut être de l'ordre de 2kg, cette valeur étant donné à titre d'exemple pour des larves d'holométaboles grégaires comme le ténébrion ou la mouche soldat, proche du stade de nymphose. Une caisse peut présenter, à vide, une masse de l'ordre de 1,5 kg. La masse d'une caisse chargée peut ainsi être typiquement de l'ordre de 3,5kg.

Typiquement, une colonne de contenants pleins peut présenter une masse totale de l'ordre de 500kg.

Les caisses présentent avantageusement une stabilité et une résistance suffisante pour résister, même empilées, aux accélérations horizontales provoquées par le dispositif automatisé permettant leur déplacement dans la première zone Z1 de l'atelier ou entre la première zone Z1 et la seconde zone Z2, et sur les éventuels moyens tel qu'un convoyeur à bande de la seconde zone Z2. En particulier, les caisses présentent avantageusement une résistance horizontale suffisante pour supporter un différentiel d'accélération entre leur face inférieure et leur face supérieure. Les caisses doivent de préférence être configurées pour pouvoir résister à une accélération horizontale comprise entre 1m/s² et 5m/s² et préférentiellement entre 3 m/s² et 4 m/s².

Les caisses présentent également avantageusement des parois latérales ajourées permettant une aération adaptée à l'élevage des insectes.

Tel que représenté à la figure 3, les contenants 31, 32 sont palettisés, c'est-à-dire groupés en unités élémentaires UE sur une palette 33 de manutention. La palette 33 peut notamment être une palette de taille classique, c'est-à-dire typiquement une palette de type « palette Europe » de 120 cm de longueur par 80 cm de largeur, ou une demi-palette de ce type, de 80 cm de longueur par 60 cm de largeur. D'autres formats de palette peuvent être employés, néanmoins l'emploi de palette de format standard permet de limiter les coûts liés à la spécialisation des équipements. Une palette en plastique alimentaire peut avantageusement être employée. Une palette métallique, par exemple en aluminium ou alliage d'aluminium, peut aussi être employée. Une palette en plastique ou métallique évite certains risques sanitaires comparativement à une palette en bois.

Dans l'exemple présenté à la figure 3, une unité élémentaire UE d'élevage comporte quatre colonnes de cinq caisses empilées. D'autres configurations sont possibles, par exemple l'empilement de plus ou moins de caisses par colonnes, d'une seule colonne de caisses, ou de deux colonnes de caisses. La forme des caisses, notamment la forme générale de leur fond, typiquement carré ou rectangulaire, peut être adaptée à la constitution souhaitée des unités élémentaires.

Par exemple, quatre piles de caisses de base rectangulaire d'environ 60 cm de longueur par 40 cm de largeur peuvent couvrir complètement une palette carrée de 120 cm de largeur. Six piles de caisses de base carrée d'environ 40 cm de côté peuvent être employées pour couvrir complètement une telle palette. Il peut également être choisi de n'y mettre que quatre piles de caisses de base carrée de 40 cm, espacées ou non entre elles. Deux piles de caisses de base rectangulaire d'environ 80 cm par 60 cm peuvent couvrir cette même une palette. Des piles de caisses de tailles différentes peuvent aussi être employées, par exemple une pile de caisses d'environ 80 cm par 60 cm et deux piles de caisses d'environ 60 cm par 40 cm.

Pour couvrir une demi-palette de 80 cm de longueur par 60 cm de largeur, on peut par exemple employer une pile de caisses de base rectangulaire de 80 cm par 60 cm environ, deux piles de caisses de 60 cm par 40 cm environ, ou quatre piles de caisses de 40 cm par 30 cm environ.

De nombreuses autres combinaisons sont envisageables.

La hauteur d'une unité élémentaire d'élevage complète peut par exemple être comprise entre 160 cm et 230 cm, et typiquement de l'ordre de 200 cm, lui permettant de se conformer à des rayonnages à palettes classiques que peut comporter la première zone Z1. Ainsi, le nombre de contenants superposés (par colonne de contenants) peut être de dix ou plus, potentiellement 15, voire plus de 25.

Une unité élémentaire peut comporter, outre une palette et des contenants, un couvercle chapeautant les contenants supérieurs (derniers contenants en haut des piles). Ce couvercle peut avoir une ou plusieurs fonctions parmi :
- la fermeture de la face supérieure des contenants supérieurs ;
- le maintien mécanique des piles, pouvant notamment être nécessaire pour la résistance des unités élémentaires aux accélérations horizontales subies lors de leurs déplacements ;
- le support d'un capteur de contrôle, tel qu'un thermomètre, un hygromètre, un capteur d'oxygène, de dioxyde de carbone,
- le support d'un dispositif d'éclairage, préférentiellement de type LED, etc.

Différentes organisations possibles de la première zone Z1 sont représentées, selon des plans schématiques, aux figures 4 et 5.

A la figure 4, la première zone Z1 comporte deux allées A1, A2, entre des rayonnages R1, R2, R3, et R4, permettant le passage respectif de deux transstockeurs T1, T2. Chaque case d'un rayonnage représente un emplacement de stockage d'une palette, ou une colonne d'emplacements de stockage. Le transstockeur T1, T2, peut se déplacer dans l'allée A1, A2 à laquelle il est associé, et prélever une palette dans l'un des emplacements des rayonnages, afin de la déplacer soit vers une interface avec la seconde zone (non représentée à la figure 4), soit vers un autre emplacement de la première zone Z1. Dans l'exemple ici représenté, une lame d'air 4 est ménagée entre certains emplacements pour palette. Cette lame d'air 4 permet d'isoler diverses parties de la première zone Z1, respectivement affectées à divers stades de croissance des insectes (ou larves, ou nymphes), nécessitant des conditions environnementales différentes. Quelle que soit l'organisation générale de la première zone Z1, plusieurs lames d'air peuvent être ménagées pour isoler plusieurs parties de ladite première zone Z1 entre elles.

Le cloisonnement de la zone Z1 en plusieurs parties ou silos permet de réduire le risque de propagation des maladies. Un silo peut être constitué, par exemple, de deux rangées de rayonnages équipées d'un transstockeur au milieu des deux rangées.

Ce cloisonnement des silos peut mettre en œuvre des lames d'air, ou tout autre moyen de cloisonnement permettant de séparer deux zones afin de pouvoir y garantir deux conditions atmosphériques (température, hygrométrie, ...) différentes et un confinement sanitaire entre les silos. Par exemple, des cloisonnements physiques peuvent être mis en œuvre. La première zone Z1 peut comporter plusieurs magasins différents, séparés par des cloisons physiques. Chaque magasin peut dans ce cas être doté d'un ou plusieurs dispositifs automatisés pour le déplacement des unités élémentaires.

Typiquement, quelle que soit l'organisation de la première zone Z1, elle peut être divisée physiquement par des lames d'air ou virtuellement en sous-zones dédiées à différents stades de maturité des insectes ou à plusieurs procédés d'élevage menés dans un élevage. Par exemple, trois procédés d'élevage peuvent être distingués, à savoir : un procédé dit de production, qui concerne l'évolution d'œufs ou de juvéniles jusqu'à un stade larvaire d'une maturité donnée pouvant correspondre au produit final de l'élevage avant éventuelle transformation, un procédé dit de reproduction, qui concerne l'évolution d'œufs ou de juvéniles jusqu'au stade de jeunes adultes, et un procédé dit de ponte, qui concerne la production d'œufs ou de juvéniles par des insectes adultes.

A la figure 5, la première zone Z1 comporte des rayonnages R1, R2, R3, et R4, et des transstockeurs T1, T2, de part et d'autre de ces rayonnages R1, R2, R3, R4. Chaque rayonnage est du type permettant une progression automatisée des palettes. Typiquement, à un étage donné d'un rayonnage R1, R2, R3, R4, un premier transstockeur T1 peut introduire une unité élémentaire d'élevage dans le rayonnage considéré. L'unité élémentaire progresse ensuite dans le rayonnage via un système motorisé ou sous l'effet de la gravité (par exemple sur un système à rouleaux). La progression peut se faire au gré du retrait par un second transstockeur T2 d'une unité élémentaire ayant progressé jusqu'à une extrémité du rayonnage opposé à l'extrémité d'introduction par le premier transstockeur. Une lame d'air 4 assure la séparation entre certaines parties de la première zone Z1, en l'occurrence entre deux séries de rayonnages.

Quelle que soit la variante de l'invention considérée, une lame d'air de l'ordre de 1m à 2m de largeur (sur toute la hauteur des rayonnages), et typiquement de 1,6m, est préférable pour isoler les parties concernées ou silos de la première zone.

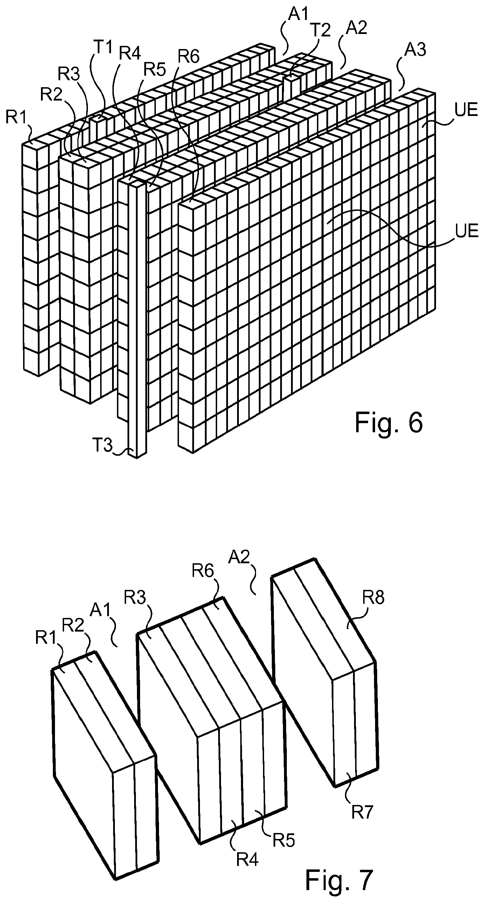

La figure 6 illustre selon une vue schématique en trois dimensions un aménagement possible d'une première zone Z1 d'un atelier conforme à un mode de réalisation de l'invention. L'organisation représentée correspond à une variante de l'organisation présentée à la figure 4, avec trois allées A1, A2, A3, entre les rayonnages R1, R2, R3, R4, R5 et R6.

Dans cette configuration à trois allées, trois transstockeurs T1, T2, T3 sont employés. Néanmoins, des transstockeurs configurés pour pouvoir desservir plusieurs allées chacun pourraient alternativement être employés.

Par ailleurs, selon ce principe général d'organisation, il est possible d'agrandir quasiment à l'infini la première zone Z1, selon l'espace disponible au sol, en agrandissant la longueur de rayonnages et/ou le nombre de rayonnages, et selon l'espace vertical disponible en augmentant la hauteur des rayonnages, ce qui permet d'accroitre considérablement la productivité de l'unité, notamment sa productivité spatiale (c'est-à-dire la production en masse rapportée à la surface au sol employée)

Dans le cas de rayonnages présentant une hauteur importante, il peut être nécessaire de garantir une circulation d'air suffisante pour homogénéiser la température dans une zone donnée (l'air chaud ayant tendance à monter en l'absence de flux organisé).

La figure 7 illustre selon une vue schématique en trois dimensions une autre variante d'organisation d'une première zone Z1 selon un mode de réalisation de l'invention. Selon cette variante, les rayonnages R1 à R8 sont organisés par groupes de deux, et chaque transstockeur pouvant se déplacer dans les allées A1, A2 est du type à double profondeur, permettant de saisir une palette ou une unité élémentaire d'élevage palettisée sur un rayonnage en seconde rangée, si l'emplacement correspondant du rayonnage en première rangée est vide. Dans d'autres variantes, le transstockeur peut permettre la récupération de palette en troisième rangée. Par ailleurs, certains transstockeurs doubles ou triples permettent la récupération simultanée de deux ou trois palettes ou unités élémentaires.

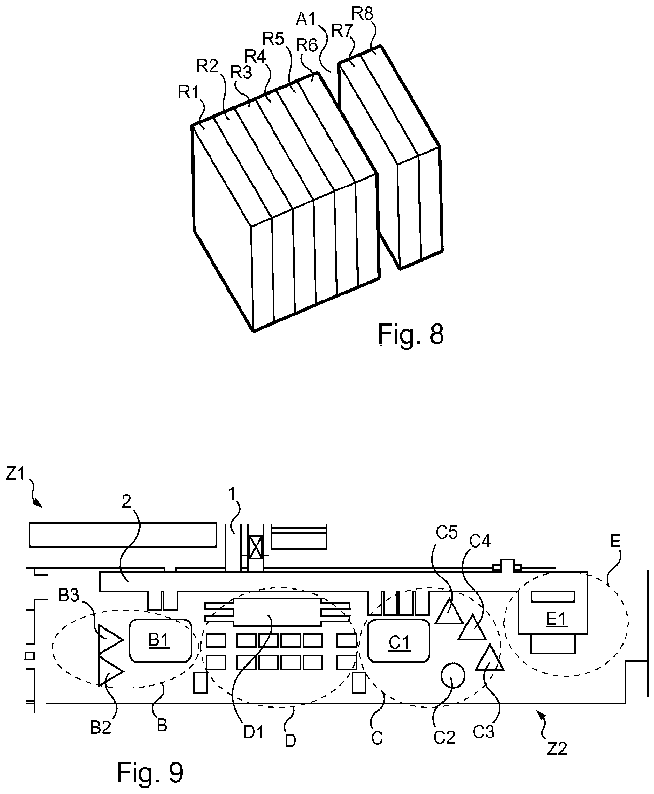

La figure 8 présente un exemple d'organisation d'une première zone d'un atelier conforme à un autre mode de réalisation de l'invention.

Dans la configuration représentée, trois à vingt rayonnages sont groupés. En l'occurrence, dans l'exemple représenté, les rayonnages R1 à R6 sont groupés. Un transstockeur est configuré pour pouvoir parcourir l'allée A1. L'allée A1 sépare les rayonnages R1 à R6 des rayonnages R7 et R8. Dans ce mode de réalisation, le transstockeur apporte un robot mobile adapté à aller chercher une unité élémentaire dans la rangée de rayonnage souhaitée du groupe de rayonnages R1 à R6, si les rangées entre l'allée A1 et la rangée souhaitée sont libres. De nombreuses variantes de ce mode de réalisation peuvent être envisagées, en modulant le nombre de rangées de rayonnages contigües, ou le nombre d'allées mises en œuvre.

Bien évidemment, les exemples présentés aux figures 4 à 8 de première zone Z1 peuvent également correspondre à l'organisation d'un seul silo d'une première zone Z1 divisée en autant de magasins physiquement cloisonnés qu'il y a de silos constitutifs de la première zone Z1.

La figure 9 présente, selon une vue schématique, un exemple d'organisation d'une seconde zone Z2 d'un atelier conforme à un mode de réalisation de l'invention.

L'exemple de seconde zone Z2 présenté à la figure 9 est représenté dans le cadre de l'exemple d'atelier de la figure 1. En particulier, on a représenté à la figure 9 l'interface 1 avec la première zone Z1. Un système de convoyage, à savoir, dans l'exemple représenté, un convoyeur à bande 2, assure le déplacement des unités élémentaires ou, le cas échéant, de contenants dégroupés. Un transstockeur, après sélection d'une palette dans la première zone Z1, dépose celle-ci sur une zone du convoyeur à bande 2 faisant interface 1 entre la première zone Z1 et la seconde zone Z2, ou tout autre dispositif permettant l'envoi au moment souhaité de la palette sur ledit convoyeur à bande 2. Dans l'exemple ici représenté, les unités élémentaires palettisées sont dirigées par le convoyeur à bande 2 vers des zones de dé-palettisation (et palettisation) en l'occurrence une première zone logistique B1 et une seconde zone logistique B2.

De manière générale, l'exemple de seconde zone Z2 ici représentée est organisée en quatre sous-zones appelées ilots, respectivement référencés B, C, D et E. Les ilots B, C, D et E sont associés à une ou plusieurs opérations d'élevage, pour lesquels ils sont plus ou moins spécialisés.

Dans l'exemple représenté, l'ilot E correspond à un poste de nourrissage des insectes (ou larves, ou nymphes). Un dispositif de nourrissage E1 équipe l'ilot de nourrissage E.

Selon diverses variantes de l'invention, le nourrissage nécessite ou non la dé-palettisation et le dégroupement des contenants formant les unités élémentaires d'élevage. La dé-palettisation peut consister à séparer chaque contenant d'une unité élémentaire les uns des autres, afin d'obtenir un ensemble de contenants individuels, ou à séparer une unité élémentaires en groupes de contenants (typiquement quatre à six contenants).

La dé-palettisation et la palettisation, dans l'ilot de nourrissage E tout comme au niveau des première et deuxième zones logistiques B1, C1, peuvent par exemple être réalisées à l'aide d'un robot de manutention poly-articulé, par exemple un robot six-axes, ou un robot sept-axes. Un tel robot peut permettre, de manière plus générale, la manipulation des contenants d'élevage avec des vitesses, des accélérations, et un maintien en position compatible de l'élevage d'insectes.

Le dispositif de nourrissage E1, que les contenants de l'unité élémentaire soient ou non dégroupés, doit assurer une répartition sensiblement uniforme de la nourriture dans les contenants.

L'ilot de nourrissage E peut optionnellement permettre un apport en eau dans les contenants d'élevage. Cet apport en eau est réalisable selon diverses modalités, alternatives ou complémentaires : par remplissage périodique d'un réservoir dédié des contenants, par brumisation ou versement, par apport d'une nourriture ou de matières riches en eau ou enrichies en eau.

Avec l'apport en eau, un apport en nutriment peut également être réalisé.

Dans l'exemple représenté, l'ilot D est spécialisé dans le lavage des contenants d'élevage. Il peut en particulier comprendre un ou plusieurs tunnels de lavage D1 adaptés pour le lavage de bacs d'élevage et/ou des palettes.

L'ilot de lavage D est, dans l'exemple ici représenté, configuré pour permettre, lorsque nécessaire, la fourniture de contenants propres à destination des ilots B et C.

Les ilots B et C correspondent, dans l'exemple représenté, à un premier ilot modulaire B et un second ilot modulaire C. Les ilots B et C sont dits modulaires en ce qu'ils comprennent un certain nombre d'équipements facilement interchangeables ou évolutifs afin de pouvoir être facilement spécialisés pour diverses opérations d'élevage. Dans la configuration représentée, les ilots modulaires B, C comprennent chacun une zone logistique B1, C1 équipée d'un robot de manutention poly-articulé, par exemple un robot six-axes, ou un robot sept-axes. Le robot équipant ces zones permet le dégroupement des contenants d'élevage lorsque cela est nécessaire pour la réalisation subséquente d'une opération d'élevage, et optionnellement le groupement et la palettisation des contenants en unités élémentaires après réalisation d'une opération d'élevage au niveau de l'ilot correspondant.

L'ilot est également configuré pour permettre la réalisation d'opérations d'élevage sur des unités élémentaires ou des contenants. L'ilot comporte ainsi un ou plusieurs postes, ou une ou plusieurs machines, vers lesquels les unités élémentaires ou les contenants doivent être envoyés. Cette fonction peut être en partie assurée par le robot de manutention, par exemple pour la dépose d'un contenant sur un convoyeur apportant l'unité élémentaire ou le contenant vers un poste donné.

Dans l'exemple représenté, le premier ilot modulaire B comporte un premier séparateur ventilateur B2, configuré pour la séparation entre larves vivantes, larves mortes, et déjections. Le premier ilot modulaire B comporte également notamment un deuxième séparateur ventilateur B3, configuré pour la calibration des larves (vivantes), c'est-à-dire la ségrégation des larves en fonction de leur taille ou de leur masse.

Dans l'exemple représenté, le second ilot modulaire C comporte un tamiseur C2, configuré pour la séparation entre insectes adultes, œufs, et substrat d'élevage (milieu ajouté dans les contenants, adapté à la vie des insectes ou larves ou nymphes). Il peut notamment s'agir d'un tamiseur à étages, les tamis successifs séparant les étages étant de plus en plus fins afin de réaliser la séparation précitée. Le second ilot modulaire C comporte également un troisième séparateur ventilateur C3, configuré pour la séparation entre insectes adultes, larves et nymphes. Le second ilot modulaire C comporte également un quatrième séparateur ventilateur C4, configuré pour la séparation entre insectes vivants et insectes morts. Le second ilot modulaire C comporte également un cinquième séparateur ventilateur C5, configuré pour la séparation entre larves et nymphes.

L'organisation de l'atelier, et en particulier de la seconde zone Z2, présentée ici à titre d'exemple, permet la réalisation de l'ensemble des opérations périodiques d'élevage d'insectes, de l'œuf jusqu'à l'obtention d'insectes adultes ayant le niveau de croissance souhaité. De nombreuses autres organisations, sont possibles, mettant en œuvre un nombre plus élevé ou moindre d'ilots ou de postes.

Un atelier conforme à l'invention est en outre avantageusement équipé d'un dispositif permettant de suivre la réalisation des différentes opérations d'élevage au cours dudit élevage. En particulier, le procédé d'élevage suit une succession d'étapes, c'est-à-dire typiquement un ordonnancement précis des opérations d'élevage réalisées selon un calendrier prédéfini, qui peut éventuellement être corrigé en cours d'élevage selon l'évolution de la croissance des insectes (ou larves ou nymphes). Afin de pouvoir suivre de manière efficace l'avancement dans le procédé d'élevage, l'atelier est avantageusement doté d'un système de suivi des unités élémentaires, et/ou de certains contenants, et/ou de chacun des contenants.

Le système de suivi des unités élémentaires, et/ou de certains contenants, et/ou de chacun des contenants peut en particulier mettre en œuvre la technologie RFID (selon l'acronyme anglophone « radio frequency identification » souvent traduit par « radio-identification »). Une étiquette RFID peut être associée, le cas échéant, aux unités élémentaires, ou contenants, des systèmes de lecture permettant leur identification étant disposés dans l'atelier, typiquement au niveau de l'interface entre la première zone et la seconde zone (pour gérer la position de l'unité élémentaire dans les rayonnages de la première zone Z1), et en entrée et/ou en sortie des différents postes auxquels sont réalisés des opérations d'élevage. Le système RFID mis en œuvre peut permettre également l'identification instantanée de l'ensemble des bacs constituant une palette. Ce système RFID est avantageusement lié à une base de données qui permet de garantir la traçabilité de chacun des contenants. La traçabilité porte sur l'ensemble du procédé d'élevage, depuis les matières premières employées pour l'élevage aux insectes (nourriture, substrat, etc.), jusqu'à l'abattage et la transformation en produits finis.

D'autres moyens d'identification et de collecte des données correspondantes peuvent être employés avec succès, par exemple une communication par ondes, notamment selon un protocole Wi-Fi, Bluetooth, ou Zigbee (marques déposées). Un système à bas débit mettant en œuvre des ondes radio basses fréquences peut aussi être mis en œuvre avec succès.

L'atelier est également avantageusement doté d'un système informatique de suivi de production, associé aux moyens de suivi du type RFID ou d'un autre type. La production peut ainsi être pilotée de manière automatisée, le système pouvant typiquement associer certaines opérations à certaines unités élémentaires d'élevage, et commander en temps voulu la récupération d'une unité élémentaire d'élevage donnée dans la première zone de stockage, la réalisation de l'opération souhaitée, et le retour à une position donnée de l'unité élémentaire.

le stade d'évolution et de croissance des insectes (œufs, larves, nymphes, insectes adultes) dans une même unité élémentaire d'élevage est identique. Pour cela, les insectes d'une même unité élémentaire sont avantageusement « synchronisés », c'est-à-dire issus d'œufs pondus à quelques jours d'intervalle au maximum, puis triés à partir du stade larvaire par taille ou par maturité. Le suivi des unités élémentaires est ainsi en général suffisant pour le pilotage des dispositifs automatisés de l'atelier, par exemple des transstockeurs pouvant être employés pour récupérer une unité dans la première zone Z1 de l'atelier et l'amener vers la seconde zone Z2 pour la réalisation d'une opération donnée, puis des dispositifs permettant l'orientation de l'unité élémentaire dans la seconde zone Z2 vers le ou les postes souhaités.

Le suivi de certains contenants particuliers peut permettre par exemple le prélèvement périodique et l'échantillonnage de ces contenants particuliers pour la réalisation de contrôles ou de prélèvements.

Enfin, le suivi individuel des contenants, nécessitant l'identification de chacun desdits contenants, permet un suivi complet et individualisé du processus d'élevage. Il permet notamment de reconstituer, au besoin, des unités élémentaires en cours d'élevage avec des contenants issus d'autres unités élémentaires ou avec de nouveaux contenants.

Un atelier d'élevage conforme à l'invention peut être employé pour l'élevage de nombreuses espèces d'insectes, moyennant de légères adaptations, typiquement dans la définition technique des contenants d'élevage, et dans la calibration des machines employées pour le nourrissage et pour les opérations de tri. Généralement, une seule espèce est élevée dans un atelier. Plusieurs espèces peuvent également être élevées, de préférence dans des parties distinctes de l'atelier. Dans le cadre d'un atelier adapté pour l'élevage simultané de plusieurs espèces d'insectes, certaines synergies peuvent être exploitées. Typiquement, certaines larves, insectes vivants ou morts, d'une espèce, ou les sous-produits de production d'un élevage, peuvent être employées pour nourrir une autre espèce.

Le ou les produit(s) d'intérêt obtenus *in fine,* après valorisation des produits de l'élevage, sont obtenus à partir d'insectes. Tel que précédemment mentionné, on entend par « insectes » des insectes à n'importe quel stade de développement, tel qu'un stade adulte, larvaire ou un stade de nymphe. De préférence, les insectes mis en œuvre dans le procédé selon l'invention sont comestibles.

Plus particulièrement, les insectes peuvent être choisis parmi le groupe constitué par les coléoptères, les diptères, les lépidoptères, les isoptères, les orthoptères, les hyménoptères, les dictyoptères, les hémiptères, les hétéroptères, les éphéméroptères et les mécoptères, de préférence, parmi les coléoptères, les diptères, les orthoptères et les lépidoptères.

Préférentiellement, les insectes sont choisis parmi le groupe constitué par *Tenebrio molitor (ou* ténébrion meunier), *Hermetia illucens, Rhynchophorus ferrugineus, Galleria mellonella, Alphitobius diaperinus, Zophobas morio, Blattera fusca, Musca domestica, Chrysomya megacephala, Locusta migratoria, Schistocerca gregaria, Acheta domesticus* et *Samia ricini.*

Des conditions favorables, notamment dans la première zone où les insectes sont stockés pendant leur croissance peuvent permettre un développement et une reproduction rapide des insectes. Par exemple, le cycle complet de développement du ténébrion meunier, de l'œuf à l'adulte à pleine croissance, peut durer de deux à trois mois à une température de 15°C à 35°C, alors qu'il peut prendre un an dans la nature.

Un atelier conforme à l'invention permet ainsi l'élevage à grande échelle d'insectes avec des coûts minimaux grâce à sa grande automatisation et à l'optimisation des dispositifs et procédés mis en œuvre. Par exemple, un transstockeur peut typiquement effectuer jusqu'à cinq cents opérations de déplacement par heure. En outre, il permet de déplacer des palettes pouvant être organisée pour porter chacune une grande quantité d'insectes. Ainsi, un tel dispositif permet d'organiser dans l'élevage un flux à très haut débit, tout en ayant une très forte densité volumique d'insecte au sein de l'élevage.

En outre, les opérations d'élevage sont effectuées dans une zone de taille limitée à des postes spécialisés et donc optimisés pour ces opérations. La croissance des insectes s'effectue dans une zone à atmosphère (température, hygrométrie...) contrôlée voire pilotée, afin de proposer des conditions de croissance optimales pour les insectes à tous les stades de l'œuf à l'adulte.

Le dispositif de contrôle peut ainsi permettre le pilotage ou régulation des paramètres environnementaux contrôlés, ou de paramètres qui y sont liés.

La zone de stockage des insectes peut en outre être particulièrement optimisée spatialement, en mettant en œuvre un stockage dans des rayonnages pouvant présenter une hauteur importante, ce qui réduit l'espace au sol nécessaire. A titre d'exemple, il est estimé qu'en employant, dans la première zone Z1, des rayonnages sur 12 mètres de hauteur, un atelier conforme à l'invention pourrait permettre la production plus de 8000 tonnes par an de protéines (en matière sèche) par hectare employé, alors que la culture de soja permet d'en produire une à cinq tonnes par hectare et par an, et un élevage de porcs ou de poulets en batteries permet de produire un équivalent de quelques dizaines de tonnes par hectare et par an.

L'atelier proposé dans l'invention permet en particulier de mettre en œuvre une méthode d'élevage fondée sur un ordonnancement séquentiel, dans deux zones distinctes, d'opérations unitaires en alternance avec des périodes « passives » de stockage pour la croissance des insectes. Une telle méthode est adaptée à la production d'insectes à échelle industrielle. A titre d'exemple, un atelier de taille modeste selon l'invention pourrait produire a minima une tonne de larves par jour, présenter une zone adaptée au stockage de cinquante tonnes d'insectes (œufs, larves, nymphes, et adultes) réparties sur 500 palettes. Les opérations d'élevage nécessitent dans ce cas le déplacement d'environ 140 palettes par jour. Grâce à l'organisation proposée dans l'invention, ces valeurs peuvent être augmentées et améliorées quasiment sans limite. Une exploitation industrielle à grande échelle, pour répondre aux besoins des marchés en alimentation animale par exemple, pourrait ainsi typiquement conduire à l'adoption de valeurs cinquante à cent fois supérieures à celles précitées, selon les marchés visés.

Enfin, l'élevage dans un atelier conforme à l'invention peut être réalisé avec des moyens et des procédés en permettant un contrôle et un suivi rigoureux, limitant les risques sanitaires dans l'élevage.

## Revendications

1. Atelier d'élevage d'insectes, comportant une première zone (Z1) dans laquelle les insectes en cours d'élevage sont stockés au cours de leur croissance dans des contenants (31, 32) et une seconde zone (Z2) comportant au moins un poste configuré pour la réalisation d'une opération d'élevage sur les insectes d'un contenant ou sur ledit contenant ;
**caractérisé en ce que** les contenants (31, 32) sont groupés dans la première zone (Z1) en ensembles de contenants (31, 32) palettisés dits unités élémentaires (UE), chaque unité élémentaire contenant lors de sa formation uniquement des insectes au même stade d'évolution, la première zone (Z1) comportant des rayonnages (R1...R8) à palettes (33) dans lesquels peuvent être disposées les unités élémentaires (UE);
la première zone (Z1) étant en outre équipée d'un dispositif automatisé configuré pour le déplacement des unités élémentaires (UE) entre la première zone (Z1) et une interface (1) avec la seconde zone (Z2), ladite interface (1) étant une zone de dépôt d'une unité élémentaire,
ladite interface étant dotée d'un système de convoyage,
le système de convoyage permettant l'envoi de l'unité élémentaire vers la seconde zone (Z2),
ou ladite interface permettant de dé-palettiser et/ou dégrouper les contenants d'élevage de l'unité élémentaire et le système de convoyage permettant leur envoi vers la seconde zone (Z2).

2. Atelier d'élevage d'insectes selon la revendication 1, dans lequel le dispositif automatisé comporte un transstockeur (T1, T2, T3) pouvant se déplacer le long de ou entre les rayonnages (R1...R8).

3. Atelier d'élevage d'insectes selon la revendication 1 ou la revendication 2, dans lequel le dispositif automatisé est adapté à se déplacer à l'intérieur des rayonnages.

4. Atelier d'élevage d'insectes selon l'une des revendication 1 à 3, dans lequel les contenants (31, 32) sont des caisses empilables, les unités élémentaires (UE) comportant une pluralité de caisses empilées, en une ou plusieurs colonnes, sur une palette (33).

5. Atelier d'élevage d'insectes selon l'une des revendications 1 à 3, dans lequel les unités élémentaires comportent un rack adapté à recevoir les contenants afin de former une ou plusieurs colonnes de contenants.

6. Atelier d'élevage d'insectes selon la revendication 4 ou la revendication 5, dans lequel une unité élémentaire (UE) comporte une à quatre colonnes constituées chacune de quatre à trente-cinq caisses.

7. Atelier d'élevage d'insectes selon l'une des revendications 1 à 6, dans lequel les unités élémentaires (UE) ont une hauteur comprise entre 1,80m et 3m, et de préférence entre 2m et 2,80m

8. Atelier d'élevage d'insectes selon l'une des revendications 1 à 7, dans lequel les rayonnages (R1...R8) sont configurés pour le stockage de deux à vingt unités élémentaires (UE) en hauteur, et de une à vingt-deux unités élémentaires (UE) en profondeur.

9. Atelier élevage d'insectes selon l'une des revendications précédentes, dans lequel la première zone (Z1) est divisée en silos, destinés au stockage de larves ou d'insectes à des stades de croissance différents et/ou d'espèces différentes, lesdits silos étant séparés par des moyens de cloisonnement

10. Atelier d'élevage d'insectes selon la revendication 8, comportant un dispositif de contrôle d'au moins un paramètre environnemental parmi la température, l'hygrométrie de l'air, la pression atmosphérique, la lumière et sa périodicité, la teneur de l'air en oxygène, la teneur de l'air en composés organiques volatils, et la teneur de l'air en particules fines, configuré pour appliquer une valeur de paramètre environnemental différente à chaque ensemble de rayonnage.

11. Atelier selon l'une des revendications précédentes, dans lequel la seconde zone (Z2) comporte un système automatisé de convoyage pour le déplacement d'unités élémentaires (UE) ou de contenants (31, 32) dégroupés vers l'au moins un poste de ladite seconde zone (Z2).

12. Atelier selon l'une des revendications précédentes, dans lequel la seconde zone (Z2) comporte un poste de dé-palettisation et dégroupage des contenants (31, 32) et dans lequel la seconde zone (Z2) comporte un poste de groupage des contenants (31, 32) en unité élémentaire.

13. Atelier selon l'une des revendications précédentes, dans lequel la seconde zone (Z2) comporte une pluralité de postes, chaque poste étant configuré pour l'une ou plusieurs opérations d'élevage choisies parmi :
- le nourrissage ;
- l'apport d'eau ;
- la calibration, en taille, masse, volume ou densité des insectes ;
- le tri entre larves vivantes, mortes, et déjections ;
- le tri entre adultes vivants et adultes morts ;
- le tri entre nymphes vivantes et nymphes mortes ;
- le tri entre au moins deux stades d'évolution des insectes entre œufs, larves, nymphes, et adultes,
- la séparation des insectes et du substrat non consommé
- le tri entre insectes et œufs ;
- l'ajout d'insectes dans un contenant d'élevage ;
- l'abattage ou la destruction d'insectes
- le lavage de contenants (31, 32).

14. Atelier selon la revendication 13, dans lequel un poste comporte un outil de vision et/ou d'échantillonnage configuré pour l'analyse de l'état physiologique des insectes, larves, nymphes.

14. Atelier selon la revendication 13, comportant un poste configuré pour la calibration, en taille, masse, volume ou densité, des insectes adultes et/ou le tri entre larves vivantes, mortes, et déjections et/ou le tri entre insectes adultes et larves ou nymphes, comportant un dispositif de séparation selon la densité et la prise à l'air.

15. Atelier selon la revendication 13 ou la revendication 14, comportant un poste configuré pour la calibration, en taille ou volume des larves vivantes et/ou le tri entre larves vivantes, mortes, insectes adultes vivants, morts, nymphes vivantes, mortes, œufs, substrat et déjections, et/ou le tri des insectes selon le stade de développement, comportant un dispositif de tri optique.

16. Atelier selon l'une des revendications 13 à 15, comportant un poste configuré pour la calibration des larves vivantes et/ou le tri entre larves vivantes, mortes, insectes adultes vivants, morts, nymphes vivantes, mortes, œufs, substrat et déjections, comportant un tamiseur, une table vibrante, ou une table densimétrique.

17. Atelier selon l'une des revendications 13 à 15, comportant un poste configuré pour la calibration des larves vivantes et/ou le tri entre larves vivantes, mortes, insectes adultes vivants, morts, nymphes vivantes, mortes, œufs, substrat et déjections, comportant une calibreuse à rouleaux.

18. Atelier selon l'une des revendications précédentes, comportant un dispositif d'identification de caisses ou d'unités élémentaires (UE) adapté à être mis en œuvre par des moyens électroniques, ledit dispositif d'indentification comportant un système de radio-identification ou un système de communication par ondes.

19. Atelier selon l'une quelconque des revendications précédentes, comportant en outre un ensemble de capteurs comportant :
- un capteur de masse configuré pour déterminer la masse d'une unité élémentaire ou d'un contenant ; et/ou
- un capteur de couleur, configuré pour déterminer la couleur des insectes, nymphe, ou œuf, du substrat, de l'eau, et/ou des déjections dans un contenant ; et/ou
- un capteur d'épaisseur ou de volume configuré pour déterminer l'épaisseur ou du volume de substrat dans un contenant ;
- un capteur de taille, configuré pour déterminer la taille des insectes, nymphe, ou œuf dans un contenant.

## Patentansprüche

1. Anlage zur Aufzucht von Insekten, enthaltend einen ersten Bereich (Z1), in dem die aufzuziehenden Insekten während ihres Wachstums in Behältern (31, 32) gelagert werden, und einen zweiten Bereich (Z2), der zumindest eine Station aufweist, die zum Ausführen eines Aufzuchtvorgangs an den Insekten eines Behälters oder an diesem Behälter ausgelegt ist;
**dadurch gekennzeichnet, dass** die Behälter (31, 32) in dem ersten Bereich (Z1) zu Sätzen von palettierten Behältern (31, 32), Grundeinheiten (UE) genannt, gruppiert sind, wobei jede Grundeinheit bei ihrer Bildung nur Insekten im gleichen Entwicklungsstadium enthält, wobei der erste Bereich (Z1) Regale (R1 ... R8) mit Paletten (33) umfasst, in denen die Grundeinheiten (UE) angeordnet werden können;
wobei der erste Bereich (Z1) ferner mit einer automatisierten Vorrichtung ausgestattet ist, die dazu ausgelegt ist, die Grundeinheiten (UE) zwischen dem ersten Bereich (Z1) und einer Schnittstelle (1) mit dem zweiten Bereich (Z2) zu verlagern, wobei die Schnittstelle (1) ein Bereich zum Ablegen einer Grundeinheit ist,
wobei die Schnittstelle über ein Fördersystem verfügt,
wobei mit dem Fördersystem die Grundeinheit in den zweiten Bereich (Z2) befördert werden kann,
oder mit der Schnittstelle die Zuchtbehälter der Grundeinheit depalettiert und/oder degruppiert werden können und das Fördersystem deren Förderung in den zweiten Bereich (Z2) ermöglicht.

2. Anlage zur Aufzucht von Insekten nach Anspruch 1,
wobei die automatisierte Vorrichtung ein Regalbediengerät (T1, T2, T3) enthält, das entlang oder zwischen Regalen (R1 ... R8) verfahrbar ist.

3. Anlage zur Aufzucht von Insekten nach Anspruch 1 oder Anspruch 2, wobei die automatisierte Vorrichtung dazu geeignet ist, sich innerhalb der Regale zu verlagern.

4. Anlage zur Aufzucht von Insekten nach einem der Ansprüche 1 bis 3,
wobei die Behälter (31, 32) stapelbare Kisten sind, wobei die Grundeinheiten (UE) eine Vielzahl von Kisten umfassen, die in einem oder mehreren Stapeln auf einer Palette (33) gestapelt sind.

5. Anlage zur Aufzucht von Insekten nach einem der Ansprüche 1 bis 3,
wobei die Grundeinheiten ein Gestell aufweisen, das dazu geeignet ist, die Behälter aufzunehmen, um einen oder mehrere Stapel von Behältern zu bilden.

6. Anlage zur Aufzucht von Insekten nach Anspruch 4 oder Anspruch 5,
wobei eine Grundeinheit (UE) einen bis vier Stapel umfasst, die jeweils aus vier bis fünfunddreißig Kisten bestehen.

7. Anlage zur Aufzucht von Insekten nach einem der Ansprüche 1 bis 6,
wobei die Grundeinheiten (UE) eine Höhe zwischen 1,80 m und 3 m, vorzugsweise zwischen 2 m und 2,80 m, haben.

8. Anlage zur Aufzucht von Insekten nach einem der Ansprüche 1 bis 7,
wobei die Regale (R1 ...R8) für die Lagerung von zwei bis zwanzig Grundeinheiten (UE) in der Höhe und von einer bis zweiundzwanzig Grundeinheiten (UE) in der Tiefe ausgelegt sind.

9. Anlage zur Aufzucht von Insekten nach einem der vorangehenden Ansprüche,
wobei der erste Bereich (Z1) in Silos unterteilt ist, die für die Lagerung von Larven oder Insekten in verschiedenen Wachstumsstadien und/oder von verschiedenen Arten bestimmt sind, wobei die Silos durch Trenneinrichtungen getrennt sind.

10. Anlage zur Aufzucht von Insekten nach Anspruch 8, enthaltend eine Vorrichtung zur Kontrolle zumindest eines Umweltparameters aus den Bereichen Temperatur, Luftfeuchtigkeit, atmosphärischer Druck, Licht und deren Periodizität, Sauerstoffgehalt der Luft, Gehalt an flüchtigen organischen Verbindungen in der Luft und Feinpartikelgehalt der Luft, die dazu ausgelegt ist, auf jede Regalanordnung einen anderen Wert für den Umweltparameter anzuwenden.

11. Anlage nach einem der vorangehenden Ansprüche,
wobei der zweite Bereich (Z2) ein automatisiertes Fördersystem zum Verlagern von degruppierten Grundeinheiten (UE) oder Behältern (31, 32) zu zumindest einer Station des zweiten Bereichs (Z2) enthält.

12. Anlage nach einem der vorangehenden Ansprüche,
wobei der zweite Bereich (Z2) eine Station zum Depalettieren und Degruppieren der Behälter (31, 32) enthält und
wobei der zweite Bereich (Z2) eine Station zum Gruppieren der Behälter (31, 32) in Grundeinheiten enthält.

13. Anlage nach einem der vorangehenden Ansprüche,
wobei der zweite Bereich (Z2) eine Vielzahl von Stationen umfasst, wobei jede Station für einen oder mehrere Aufzuchtvorgänge ausgelegt ist, die ausgewählt sind aus:
- Fütterung;
- Wasserversorgung;
- Kalibrierung in Bezug auf Größe, Masse, Volumen oder Dichte der Insekten;
- Aussortierung zwischen lebenden und toten Larven und Exkrementen;
- Aussortierung zwischen adulten lebenden und toten Insekten;
- Aussortierung zwischen lebenden Nymphen und toten Nymphen;
- Aussortierung zwischen zumindest zwei Entwicklungsstadien der Insekten zwischen Eiern, Larven, Nymphen und adulten Insekten;
- Trennung von Insekten und unverbrauchtem Substrat;
- Aussortierung zwischen Insekten und Eiern;
- Hinzufügung von Insekten in einen Zuchtbehälter;
- Töten oder Vernichten von Insekten;
- Reinigung von Behältern (31, 32).

14. Anlage nach Anspruch 13, wobei eine Station ein Beobachtungs- und/oder Probenahme-Instrument enthält, das zur Analyse des physiologischen Zustands der Insekten, Larven und Nymphen ausgelegt ist.

14. Anlage nach Anspruch 13, enthaltend eine Station, die zur Kalibrierung in Bezug auf Größe, Masse, Volumen oder Dichte von adulten Insekten und/oder zur Aussortierung zwischen lebenden und toten Larven und Exkrementen und/oder zur Aussortierung zwischen adulten Insekten und Larven oder Nymphen ausgelegt ist, enthaltend eine Vorrichtung zum Trennen nach Dichte und Luftzufuhr.

15. Anlage nach Anspruch 13 oder Anspruch 14, enthaltend eine Station, die zur Kalibrierung in Bezug auf Größe bzw. Volumen von lebenden Larven und/oder zur Aussortierung zwischen lebenden und toten Larven, lebenden und toten adulten Insekten, lebenden und toten Nymphen, Eiern, Substrat und Exkrementen und/oder zur Aussortierung von Insekten nach dem Entwicklungsstadium ausgelegt ist, enthaltend eine optische Sortiervorrichtung.

16. Anlage nach einem der Ansprüche 13 bis 15, enthaltend eine Station, die zur Kalibrierung lebender Larven und/oder zur Aussortierung zwischen lebenden und toten Larven, lebenden und toten adulten Insekten, lebenden und toten Nymphen, Eiern, Substrat und Exkrementen ausgelegt ist, enthaltend eine Siebeinrichtung, einen Rütteltisch oder einen Dichtetisch.

17. Anlage nach einem der Ansprüche 13 bis 15, enthaltend eine Station, die zur Kalibrierung lebender Larven und/oder zur Aussortierung zwischen lebenden und toten Larven, lebenden und toten adulten Insekten, lebenden und toten Nymphen, Eiern, Substrat und Exkrementen ausgelegt ist, enthaltend einen Rollensortierer.

18. Anlage nach einem der vorangehenden Ansprüche, enthaltend eine Vorrichtung zur Identifizierung von Kisten oder Grundeinheiten (UE), die dazu geeignet ist, über elektronische Mittel betrieben zu werden, wobei die Identifizierungsvorrichtung ein Funk-Identifizierungssystem oder ein Wellenkommunikationssystem aufweist.

19. Anlage nach einem der vorangehenden Ansprüche, ferner enthaltend eine Anordnung von Sensoren, enthaltend :
- einen Massensensor, der dazu ausgelegt ist, die Masse einer Grundeinheit oder eines Behälters zu bestimmen; und/oder
- einen Farbsensor, der dazu ausgelegt ist, die Farbe von Insekten, Nymphen oder Eiern, Substrat, Wasser und/oder Exkrementen in einem Behälter zu bestimmen; und/oder
- einen Dicken- bzw. Volumensensor, der dazu ausgelegt ist, die Dicke bzw. das Volumen des Substrats in einem Behälter zu bestimmen;
- einen Größensensor, der dazu ausgelegt ist, die Größe von Insekten, Nymphen oder Eiern in einem Behälter zu bestimmen.

## Claims

1. Farm for rearing insects, comprising a first zone (Z1) in which the insects being raised are stored during their growth in containers (31, 32) and a second zone (Z2) comprising at least one station configured for performing a raising operation on the insects of a container or on said container;
**characterized in that** the containers (31, 32) are grouped in the first zone (Z1) in palletized sets of containers (31, 32) referred to as basic units (UE), each elementary unit containing, when formed, only insects at the same stage of development, the first zone (Z1) comprising racks (R1 ... R8) for pallets (33) in which may be disposed the basic units (UE);
the first zone (Z1) being furthermore equipped with an automatic device configured for the movement of the basic units (UE) between the first zone (Z1) and an interface (1) with the second zone (Z2), said interface being a zone for depositing a basic unit
said interface being equipped with a conveyor system,
said conveyor system enabling the sending of the basic unit to the second zone (Z2),
or said interface enabling to de-palletize and/or to ungroup the rearing containers from the basic unit and the conveyor system enabling to send them to the second zone (Z2).

2. Farm for rearing insects according to claim 1, in which the automatic device comprises a storage and retrieval machine (T1, T2, T3) able to move along or between the racks (R1 ... R8).

3. Farm for rearing insects according to claim 1 or claim 2, in which the automatic device is adapted to move within the racks.

4. Farm for rearing insects according to one of claims 1 to 3, in which the containers (31, 32) are stackable crates, the basic units (UE) comprising a plurality of stacked crates, in one or more columns, on a pallet (33).

5. Farm for rearing insects according to one of claims 1 to 3, in which the basic units comprise stand configured to receive the containers in order to form one or more columns of containers.

6. Farm for rearing insects according to claim 4 or claim 5, in which a basic unit (UE) comprises one to four columns each constituted by four to thirtyfive crates.

7. Farm for rearing insects according to one of claims 1 to 6, in which the basic units (UE) have a height comprised between 1.80m and 3m, and preferably between 2m and 2.80m.

8. Farm for rearing insects according to one of claims 1 to 7, in which the racks (R1 ... R8) are configured for the storage of two to twenty basic units (UE) in height, and of one to twenty-two basic units (UE) in depth.

9. Farm for rearing insects according to one the preceding claims, in which the first zone (Z1) is divided into silos, for the storage of larvae or insects at different stages of growth and/or of different species, said silos being separated by partitioning means.

10. Farm for rearing insects according to claim 8 comprising a device for controlling at least one environmental parameter from among temperature, air humidity, atmospheric pressure, light and its periodicity, the oxygen content of the air, the organic volatile content of the air, and the particulate content of the air, which is configured to apply a different environmental parameter value to each rack set.

11. Farm according to one of the preceding claims, in which the second zone (Z2) comprises an automatic conveying system for the movement of ungrouped containers (31, 32) or basic units (UE) to the at least one station of the second zone (Z2).

12. Farm according to one of the preceding claims, in which the second zone (Z2) comprises a de-palletising and ungrouping station for the containers (31, 32) and in which the second zone (Z2) comprises a station for grouping containers (31,32) by basic unit.

13. Farm according to one of the preceding claims, in which the second zone (Z2) comprises a plurality of stations, each station being configured for one or more rearing operations chosen from:
- feeding;
- providing water;
- calibrating, by size, mass, volume or density of the insects;
- sorting between living larvae, dead larvae and dejections;
- sorting between living adults and dead adults;
- sorting between living nymphs and dead nymphs;
- sorting between at least two stages of development of insects between eggs, larvae, nymphs, and adults;
- separating the insects from the unconsumed raising medium;
- sorting between insects and eggs;
- adding insects into a rearing container;
- killing or destroying insects;
- washing containers (31,32).

14. Farm according to claim 13, wherein a station comprises a vision and/or sampling tool configured for analysing the physiological condition of insects, larvae, nymphs.

14. Farm according to claim 13, comprising a station configured for calibrating adult insects by size, mass volume or density, or for sorting between living larvae, dead larvae, and dejections and/or for sorting between adult insects and larvae or nymphs, comprising a device for separating according to density and air resistance.

15. Farm according to claim 13 or claim 14, comprising a station configured for calibrating, by size or volume of living larvae, and/or for sorting between living larvae, dead larvae, living adult insects, dead adult insects, living nymphs, dead nymphs, eggs, raising medium and dejections, and/or the sorting of insects according to the stage of development, comprising an optical sorting device.

16. A farm according to one of claims 13 to 15, comprising a station configured for calibrating living larvae, and/or for sorting between living larvae, dead larvae, living adult insects, dead adult insects, living nymphs, dead nymphs, eggs, raising medium and dejections, comprising a screen, a vibrating table, or a densimetric table.

17. A farm according to one of claims 13 to 15, comprising a station configured for calibrating living larvae, and/or for sorting between living larvae, dead larvae, living adult insects, dead adult insects, living nymphs, dead nymphs, eggs, raising medium and dejections, comprising a roller grader.

18. A farm according to one of the preceding claims, comprising an identifying device for identifying crates or basic units (UE) adapted to be implemented by electronic means, said identifying device comprising a radiofrequency identification system or a wave communication system.

19. A farm according to any one of the preceding claims, further comprising a set of sensors comprising:
- a mass sensor configured to determine the mass of a basic unit or of a container; and/or
- a color sensor, configured to determine the color of the insects, nymph, or egg, of the raising medium, of the water, and/or of the dejections in a container; and/or
- a sensor of thickness or of volume configured to determine the thickness or the volume of raising medium in a container;
- a size sensor, configured to determine the size of the insects, nymph, or egg in a container.
